# FASCICULE DE BREVET EUROPEEN

(11) **EP 3 221 290 B1**
(45) Date de publication et mention de la délivrance du brevet: **17.07.2019**
(21) Numéro de dépôt: 15804899.1
(22) Date de dépôt: 18.11.2015
(51) Int. Cl.: C07C 61/04, C11B 9/00

(54) **ACIDE 2-OCTYLCYCLOPROPYL-1-CARBOXYLIQUE ET SES ISOMÈRES ET UTILISATIONS**
2-OCTYLCYCLOPROPYL-1-CARBONSÄURE UND ISOMERE DAVON SOWIE VERWENDUNGEN DAVON
2-OCTYLCYCLOPROPYL-1-CARBOXYLIC ACID AND THE ISOMERS THEREOF, AND USES OF SAME

(30) Priorité: 19.11.2014 FR 1461213
(43) Date de publication de la demande: 27.09.2017
(73) Titulaire: Albert, Vieille, 06220 Vallauris (FR); Centre National de la Recherche Scientifique, 75794 Paris Cedex 16 (FR); Université de Nice, 06103 Nice Cedex 2 (FR)
(72) Inventeur: BALDOVINI, Nicolas, 06200 Nice (FR); CERUTTI-DELASALLE, Céline, 06130 Grasse (FR)
(74) Mandataire: Santarelli
(86) Numéro de dépôt international: PCT/FR2015/053123
(87) Numéro de publication internationale: WO 2016/079431

(56) Documents cités:
- SU-A1- 406 543
- US-A- 4 202 902
- US-A1- 2011 263 725

## Description

L'invention concerne le domaine de la parfumerie et des composés chimiques qui constituent l'odeur de l'encens.

L'encens, appelé également oliban, est une résine aromatique produite à partir de la résine d'arbres du genre Boswellia. Cette oléorésine contient des sucres, des alcools et des acides.

Seul l'arbre mâle produit la précieuse résine, mais il faut attendre une bonne dizaine d'années pour qu'il fournisse un produit de qualité.

En parfumerie proprement dite, l'encens est utilisé sous forme d'huile essentielle ou d'absolue. C'est une essence à l'odeur fine et complexe, utilisée pour son odeur boisée, résineuse, balsamique, fraîche, assez minérale, aux aspects légèrement épicés.

De nombreux parfums sur le marché contiennent de l'huile essentielle d'oliban comme ingrédient olfactif clé.

À l'heure actuelle, le seul matériau odorant synthétique présentant cette note typique d'encens est le Mystikal®, de la société Givaudan.

Le document US 2011/0263725 décrit l'acide 2-méthylundécanoïque comme composé odorant présentant une odeur d'encens oliban.

Il existe donc un besoin en molécules synthétiques capables d'évoquer l'encens.

Les études conduites par la demanderesse sur différents extraits, fractions, et dérivés de l'oliban ont permis d'identifier plusieurs des principaux composés d'impact olfactif.

Parmi ces molécules d'impact, les acides cyclopropanecarboxyliques occupent une place particulière, notamment les acides 2-octylcyclopropyl-1-carboxyliques de formule générale (I) : 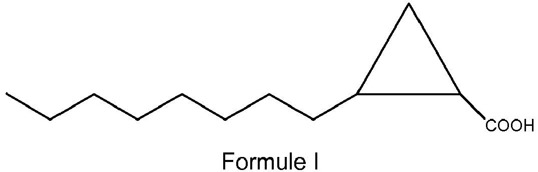 qui peuvent être sous la forme de 4 isomères de formules II, III, IV ou V.

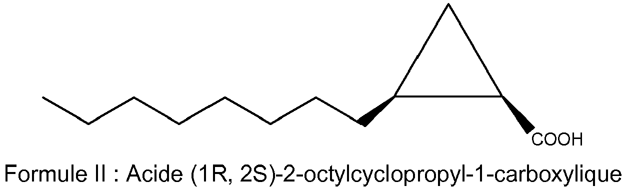

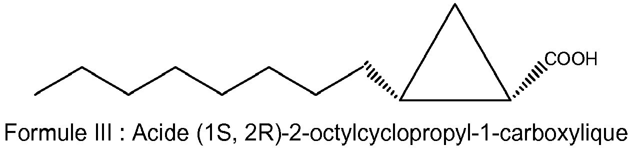

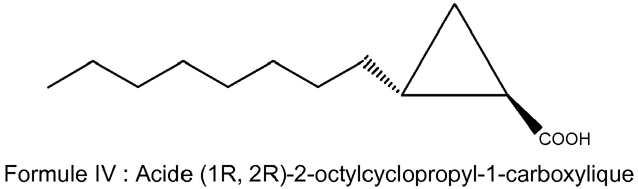

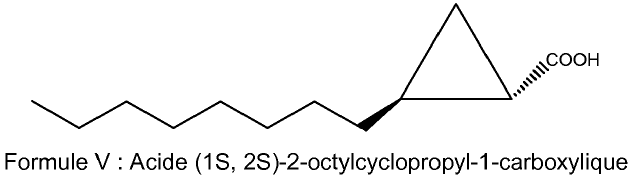

Le document SU406543 décrit l'utilisation des acides cyclopropanecarboxyliques comme antifongique.

Le document US 4,202,902 décrit l'utilisation des acides cyclopropanecarboxyliques comme inhibiteur de la lipogenèse chez les mammifères.

En effet, les travaux d'analyse réalisés par la demanderesse ont montré que ces substances participaient de manière cruciale à la note de fond de l'essence d'oliban, caractéristique de l'odeur de "vieille église".

Ainsi l'invention a pour objet premier les acides 2-octylcyclopropyl-1-carboxyliques de formule général I, sous forme d'isomères cis de formules II ou III (acide (1R, 2S)-2-octylcyclopropyl-1-carboxylique et acide (1S, 2R)-2-octylcyclopropyl-1-carboxylique) ou trans de formules IV ou V (acide (1R, 2R)-2-octylcyclopropyl-1-carboxylique et acide (1S, 2S)-2-octylcyclopropyl-1-carboxylique) isolés ou d'un mélange en toutes proportions des isomères cis de formules II ou III et/ou trans de formules IV et V .

On comprend donc que l'invention vise aussi bien les mélanges en toutes proportions
- Isomères de formule II, III et IV,
- Isomères de formule II, III et V,
- Isomères de formule II, IV et V,
- Isomères de formule III, IV et V,
- Isomères de formule II et III,
- Isomères de formule II et IV
- Isomères de formule II et V,
- Isomères de formule III et IV,
- Isomères de formule III et V,
   ainsi que les isomères de formules II, ou III ou IV ou V sous forme isolée, c'est-à-dire purs.

Par en toute proportion on comprend que chacun des composés de formules II, ou III ou IV ou V peut être dans le mélange en une proportion comprise de 0,00000001% à 99,99999999%, la quantité totale des composés dans ledit mélange pouvant représenter jusqu'à 100% dudit mélange. Par simplification et pour alléger le présent texte le titulaire ne va pas énumérer l'ensemble des combinaisons possibles mais il est bien entendu que la présente demande vise toutes ces combinaisons.

Par isolé on entend que ledit composé a subi au moins une étape de purification et que ledit composé est seul sans aucune trace des autres composés. Par exemple un composé de formule II isolé est un composé de formule II ayant subi au moins une étape de purification et présent seul sans trace des composés de formules III, IV ou V, c'est-à-dire que le composé considéré est à 100%.

L'invention a également pour objet une composition comprenant au moins de l'acide 2-octylcyclopropyl-1-carboxylique de formule générale (I). Par composition on entend un mélange du ou des composés en toutes proportions selon l'invention et d'au moins un autre ingrédient, différents des composés II, III, IV ou V. Une composition ici n'est donc pas l'un des composés isolé pur mais peut être un mélange de l'un des composés isolé pur avec un solvant comme de l'eau par exemple.

Selon une première forme de l'invention la composition peut comprendre de l'acide 2-octylcyclopropyl-1-carboxylique de formule générale (I) sous la forme de l'un quelconque des mélanges des isomères décrits ci-dessus mais également sous la forme de l'un quelconque des isomères de formule II, III, IV ou V, isolé.

Selon l'invention, ladite composition peut comprendre l'acide 2-octylcyclopropyl-1-carboxylique de formule générale (I) sous la forme de l'un quelconque des mélanges des isomères décrits ci-dessus ou sous la forme de l'un quelconque des isomères de formule II, III, IV ou V, isolé en une quantité comprise entre 0,00000001% à 99,99999999%, en poids de la composition, préférentiellement entre 0,00000005% et 50% en poids de la composition, encore plus préférentiellement entre 0,0000001% et 10%.

Selon l'invention, ladite composition peut être une composition cosmétique, pharmaceutique, vétérinaire, phytosanitaire, hygiénique, de nettoyage, ou de lavage. L'invention a encore pour objet un procédé de synthèse l'acide 2-octylcyclopropyl-1-carboxylique de formule générale (I).

L'invention a encore pour objet l'utilisation de l'acide 2-octylcyclopropyl-1-carboxylique de formule générale (I) à titre d'agent parfumant.

Selon l'invention l'acide 2-octylcyclopropyl-1-carboxylique de formule générale (I) pourra être utilisé, à titre d'agent parfumant, sous la forme de l'un quelconque des mélanges des isomères décrits ci-dessus mais également sous la forme de l'un quelconque des isomères de formule II, III, IV ou V, isolé.

Avantageusement l'agent parfumant pourra être destiné à entrer dans tout type de compositions connues de l'homme du métier comme par exemple un parfum, une eau de parfum, une eau de toilette, des produits d'hygiène, des produits cosmétiques, des savons, des lessives ou encore des bâtonnets d'encens ou des bougies.
Figure 1 : schéma réactionnel de la synthèse des formes racémiques des isomères cis-et trans-de l'acide 2-octylcyclopropyl-1-carboxylique.
Figure 2 : schéma réactionnel de la synthèse de chacun des énantiomères de l'acide 2-octylcyclopropyl-1-carboxylique [-9].
Figure 3 : Profils de chaque énantiomère (8 - 9) de l'acide 2-octylcyclopropyl-1-carboxylique obtenus par GC-MS énantiosélective (Es).

D'autres caractéristiques et avantages de l'invention ressortiront des exemples qui suivent, donnés à titre illustratif et non limitatif ainsi que de :

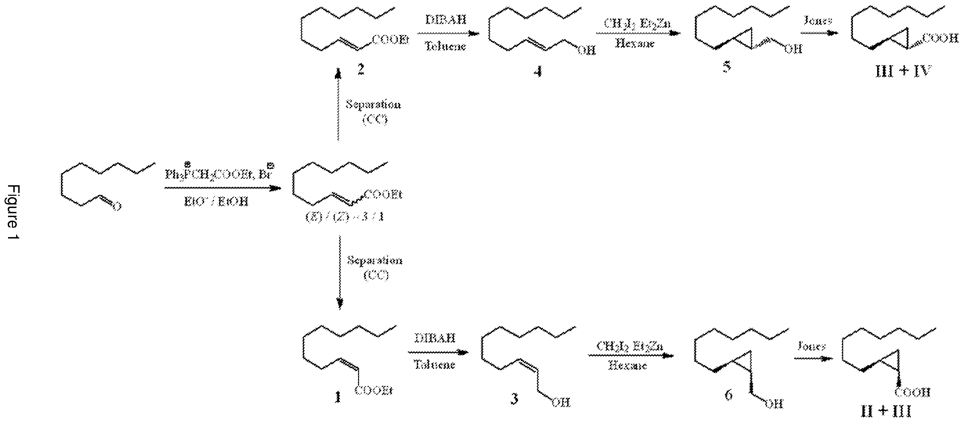

- la figure 1 qui représente le schéma réactionnel de la synthèse des formes racémiques des isomères cis-et trans-de l'acide 2-octylcyclopropyl-1-carboxylique décrite aux exemples ci-après.

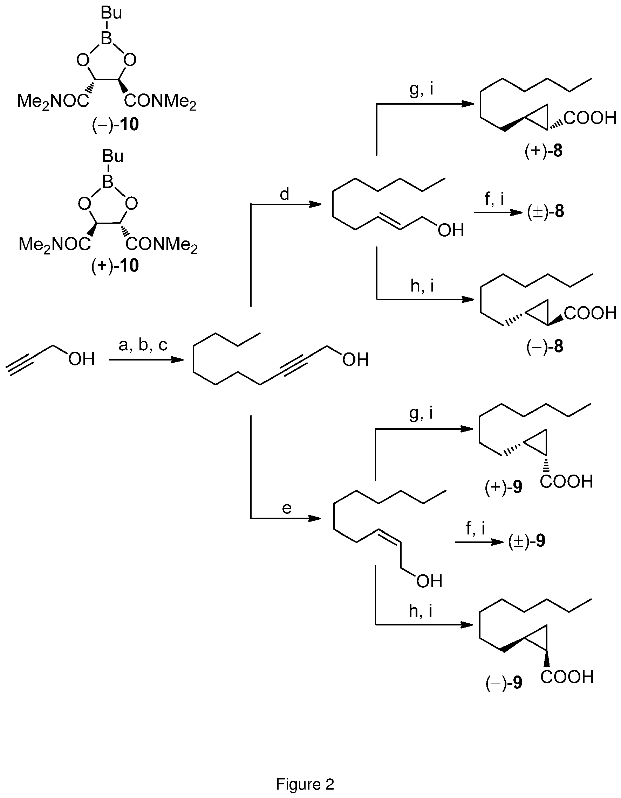

- La figure 2 qui représente le schéma réactionnel de la synthèse de chacun des énantiomères de l'acide 2-octylcyclopropyl-1-carboxylique [-9]

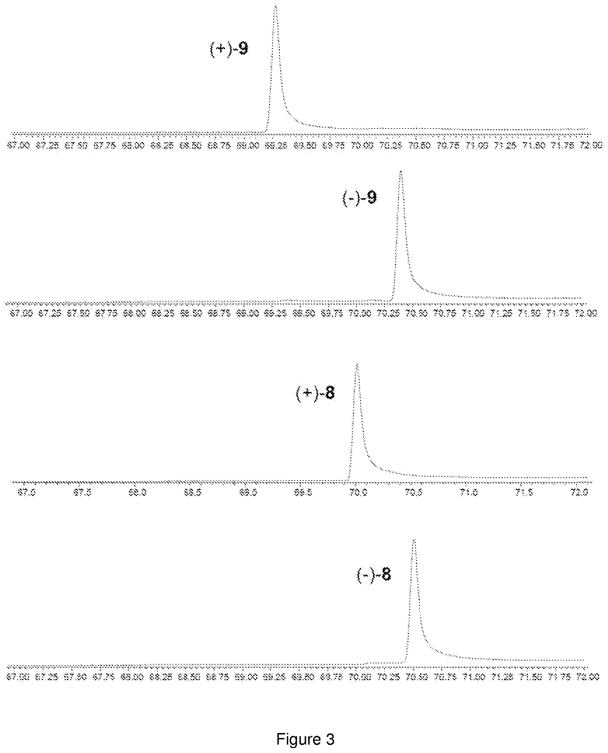

- La figure 3 qui représente les profils de chaque énantiomère de l'acide 2-octylcyclopropyl-1-carboxylique obtenus, réalisés par GC-MS énantiosélective (Es) avec
   ∘ [(+)-9] : profil de l'acide (+)-*cis*-2-octylcyclopropyl-1-carboxylique ;
   ∘ [(-)-9] : profil de l'acide (-)-*cis*-2-octylcyclopropyl-1-carboxylique ;
   ∘ [(+)-8] : profil de l'acide (+)-trans-2-octylcyclopropyl-1-carboxylique ;
   ∘ [(-)-8] : profil de l'acide (-)-*trans*-2-octylcyclopropyl-1-carboxylique.

### Exemple - synthèse des formes racémiques des isomères CIS-et TRANS-de l'acide 2-octylcyclopropyl-1-carboxylique

### Exemple 1 : Synthèse du (Z)-et du (E)-undec-2-énoate d'éthyle (1 et 2) :

A une solution d'éthylate de sodium (préparée en dissolvant du sodium (3,0 g, 130 mmol, 1,2 eq.) dans 180 ml d'éthanol absolu), on ajoute du bromure d'éthoxycarbonylmethyltriphenylphosphonium (53,40 g, 126,5 mmol, 1,2 eq.) et le mélange résultant est agité pendant 20 min.

Du nonanal (14,98 g, 105 mmol, 1 eq.) est alors ajouté, et le mélange résultant est agité pendant 20h à température ambiante, puis évaporé.

Après évaporation, une huile jaune est obtenue et les 2 composés sont purifiés par chromatographie sur colonne de silice (éther de pétrole / diéthylether (98/2)) pour donner le (Z)-undec-2-énoate d'éthyle (**1**) (2,66 g, 12%) et le (E)-undec-2-énoate d'éthyle (**2**) (4,54 g, 27%).

### Analyses spectroscopiques :

### (Z)-undec-2-énoate d'éthyle (1) :

### RMN 1H (200 MHz, CDCl3) :

δ = 6,22 (dt, J = 11,5 Hz, J = 7,5 Hz, 1 H), 5,77 (dt, J = 11,5 Hz, J = 1,7 Hz, 1 H), 4,16 (q, J = 7,2 Hz, 2 H), 2,62 (qd, J = 7,6 Hz, J = 1,7 Hz, 2 H), 1,20-1,50 (m, 12 H), 1,29 (t, J = 7,1 Hz, 3 H), 0,88 (t, J = 6,6 Hz, 3 H) ppm.

### RMN 13C (50 MHz, CDCl3) :

δ = 166,52, 150,70, 119,67, 59,77, 31,96, 29,50, 29,42, 29,35, 29,15, 29,08, 22,76, 14,33, 14,16 ppm.

### MS (El, 70 eV) :

212 (M+, 5), 167 (39), 127 (95), 115 (27), 101 (24), 99 (100), 88 (27), 81 (34), 55 (43), 43 (33), 41 (37).

### (E)-undec-2-énoate d'éthyle (2) :

### 1H NMR (200 MHz, CDCl3):

δ = 6,92 (dt, J = 15,6 Hz, J = 7,0 Hz, 1 H), 5,77 (dt, J = 15,6 Hz, J = 1,5 Hz, 1 H), 4,13 (q, J = 7,2 Hz, 2 H), 2,15 (qd, J = 7,3 Hz, J = 1,1 Hz, 2 H), 1,15-1,50 (m, 12 H), 1,29 (t, J = 7,1 Hz, 3 H), 0,88 (t, J = 6,6 Hz, 3 H) ppm.

### 13C NMR (50 MHz, CDCl3) :

δ = 166,73, 149,42, 121,28, 60,08, 32,24, 31,90, 29,41, 29,25, 29,21, 28,08, 22,70, 14,29, 14,10 ppm.

### MS (El, 70 eV) :

212 (M+, 1), 167 (93), 124 (55), 101 (100), 99 (45), 88 (44), 84 (40), 81 (45), 73 (60), 55 (74), 43 (40), 41 (48).

### Exemple 2 : synthèse du (Z)-undec-2-èn-1-ol (3) :

A une solution de (Z)-undec-2-énoate d'éthyle (1) (2,48 g, 11,7 mmol, 1 eq.) dans le toluène (70 ml), on ajoute goutte à goutte une solution 1M d'hydrure de diisobutylaluminium (DIBALH : 33 ml, 33 mmol, 2,8 eq.) dans le cyclohexane à -65°C. Le mélange réactionnel est laissé revenir lentement à température ambiante et est agité pendant 13h.

Il est ensuite à nouveau refroidi à -65°C, additionné goutte à goutte d'une solution d'acide acétique à 50% (6,5 ml) puis laissé revenir lentement à température ambiante sous agitation.

25 g de sulfate de sodium anhydre sont ensuite ajoutés et le mélange est agité pendant 1h, puis filtré sur Celite.

Les filtrats sont évaporés pour donner une huile jaune (2,03 g) qui est alors purifiée par chromatographie sur colonne de silice (éther de pétrole / diéthylether (95/5→ 9/1)) pour donner le (Z)-undec-2-èn-1-ol (3) (1,58 g, 79%).

### Analyses spectroscopiques :

### RMN 1H (200 MHz, CDCl3) :

δ = 5,55 (m, 2 H), 4,17 (d, J = 5,4 Hz, 2 H), 2,05 (q, J = 6,1 Hz, 2 H), 1,20-1,45 (m, 12 H), 0,88 (t, J = 6,7 Hz, 3 H) ppm.

### RMN 13C (50 MHz, CDCl3) :

δ = 133,27, 128,43, 58,65, 31,99, 29,73, 29,57, 29,39, 29,35, 27,54, 22,78, 14,21 ppm.

### MS (El, 70 eV) :

170 (M+, <1), 95 (28), 82 (43), 81 (31), 69 (27), 68 (33), 67 (34), 57 (100), 55 (41), 43 (43), 41 (50).

### Exemple 3 : synthèse du (E)-undec-2-èn-1-ol (4) :

Le même procédé que celui qui est appliqué pour la synthèse du (Z)-undec-2-èn-1-ol (3) (exemple 2) est appliqué au (E)-undec-2-énoate d'éthyle (2) pour conduire au (E)-undec-2-èn-1-ol (4) (85%).

### Analyses spectroscopiques :

### RMN 1H (200 MHz, CDCl3) :

δ = 5,48-5,74 (m, 2 H), 4,02 (d, J = 4,6 Hz, 2 H), 2,41 (br, s, 1 H), 2,00 (q, J = 6,9 Hz, 2 H), 1,15-1,40 (m, 12 H), 0,85 (t, J = 6,2 Hz, 3 H) ppm.

### RMN 13C (50 MHz, CDCl3) :

δ = 133,32, 128,92, 63,60, 32,30, 31,96, 29,55, 29,36, 29,29, 29,24, 22,73, 14,14. ppm.

### MS (El, 70 eV) :

170 (M+, <1), 82 (43), 81 (34), 69 (32), 68 (36), 67 (41), 57 (100), 55 (51), 54 (33), 43 (49), 41 (63).

### Exemple 4 : synthèse du trans-2-octylcyclopropyl-1-methanol (5) :

A une solution de (E)-undec-2-èn-1-ol (4) (332 mg, 1,95 mmol, 1 eq.) dans l'hexane (10 ml), on ajoute goutte à goutte une solution de diéthylzinc (10 ml, 10 mmol, 5,1 eq.) dans l'hexane at -50°C, puis du diiodométhane (5,28 g, 19,7 mmol, 10 eq.).

Le mélange réactionnel est laissé revenir lentement à température ambiante et est agité pendant 18h. Il est ensuite à nouveau refroidi à -40°C, additionné goutte à goutte d'une solution de chlorure d'ammonium (20 ml) puis laissé revenir lentement à température ambiante.

Le mélange est alors décanté, et les phases aqueuses sont réextraites avec 2×25 ml de diéthyléther.

Les phases organiques sont rassemblées, lavées à la saumure, puis séchées sur sulfate de magnésium et évaporées pour donner une huile orange (788 mg) qui est alors purifiée par chromatographie sur colonne de silice (éther de pétrole / diéthylether (98/2→ 8/2)) pour donner le trans-2-octylcyclopropyl-1-methanol (5) (275 mg, 77%).

### Analyses spectroscopiques :

### RMN 1H (200 MHz, CDCl3) :

δ = 3,28-3,48 (m, 2 H), 2,27 (br, s, 1 H), 1,10-1,40 (m, 14 H), 0,88 (t, J = 6,3 Hz, 3 H), 0,70-0,85 (m, 1 H), 0,45-0,63 (m, 1 H), 0,18-0,38 (m, 2 H) ppm.

### RMN 13C (50 MHz, CDCl3) :

δ = 67,06, 33,71, 31,98, 29,71, 29,67, 29,54, 29,41, 22,74, 21,14, 17,20, 14,15, 9,99 ppm.

### MS (El, 70 eV) :

143 (34), 83 (67), 82 (38), 81 (41), 69 (100), 68 (34), 67 (46), 57 (66), 55 (70), 43 (49), 41 (64).

### Exemple 5 : Synthèse du cis-2-octylcyclopropyl-1-methanol (6) :

Le même procédé que celui qui est appliqué pour la synthèse du trans-2-octylcyclopropyl-1-methanol (5) est appliqué au (Z)-undec-2-èn-1-ol (3) pour conduire au cis-2-octylcyclopropyl-1-methanol (6) (64%).

### Analyses spectroscopiques :

### RMN 1H (200 MHz, CDCl3) :

δ = 3,47-3,74 (m, 2 H), 1,00-1,48 (m, 16 H), 0,87 (t, J = 6,3 Hz, 3 H), 0,62-0,78 (m, 1 H), -0,10-0,01 (q, 1 H) ppm.

### MS (El, 70 eV) :

143 (30), 83 (66), 82 (35), 81 (39), 69 (100), 68 (30), 67 (44), 57 (56), 55 (65), 43 (45), 41 (59).

### Exemple 6 : Synthèse du mélange des acides trans-2-octylcyclopropyl-1-carboxylique [Acide (1S, 2R)-2-octylcyclopropyl-1-carboxylique (III) + acide (1R, 2R)-2-octylcyclopropyl-1-carboxylique (IV)] :

A une solution de trans-2-octylcyclopropyl-1-methanol (5) (130 mg, 0,71 mmol, 1 eq.) dans l'acétone (3 ml), on ajoute goutte à goutte une solution de trioxyde de chrome (322 mg, 3,22 mmol, 4,6 eq.) dans l'acide sulfurique à 36%, à 0°C.

Le mélange réactionnel est agité pendant 1h, puis de l'eau (5 ml) est ajoutée et le mélange est évaporé, additionné de 25 ml d'eau supplémentaire, puis extrait avec 2×125 ml de dichlorométhane.

Les phases organiques sont combinées, lavées à la saumure, puis séchées sur sulfate de magnésium et évaporées pour donner une huile qui est alors purifiée par chromatographie sur colonne de silice [éther de pétrole / diéthylether (9/1→ 8/2)] pour donner le mélange de l'acide (1S, 2R)-2-octylcyclopropyl-1-carboxylique (III) et de l'acide (1R, 2R)-2-octylcyclopropyl-1-carboxylique (IV) (141 mg, 73%).

### Analyses spectroscopiques :

### RMN 1H (200 MHz, CDCl3) :

δ = 11,73 (br, s, 1 H), 1,14-1,48 (m, 17 H), 0,88 (t, J = 6,3 Hz, 3 H), 0,70-0,82 (m, 1 H) ppm.

### RMN 13C (50 MHz, CDCl3) :

δ = 181,54, 33,18, 32,02, 29,67, 29,42, 29,41, 29,19, 24,19, 22,81, 20,27, 16,52, 14,23 ppm.

### MS (El, 70 eV) :

180 (1), 151 (5), 138 (15), 97 (39), 84 (34), 83 (39), 73 (100), 70 (35), 69 (51), 56 (46), 55 (79), 43 (66), 41 (72).

### Exemple 7 : Synthèse du mélange des acides cis-2-octylcyclopropyl-1-carboxylic [acide (1R, 2S)-2-octylcyclopropyl-1-carboxylique (II) et acide (1S, 2R)-2-octylcyclopropyl-1-carboxylique (III)] :

Le même procédé que celui qui est appliqué pour la synthèse du mélange des acides trans-2-octylcyclopropyl-1-carboxylique [Acide (1S, 2R)-2-octylcyclopropyl-1-carboxylique (III) + acide (1R, 2R)-2-octylcyclopropyl-1-carboxylique (IV)] (exemple 6) est appliqué au cis-2-octylcyclopropyl-1-methanol (6) pour conduire au mélange des acides cis-2-octylcyclopropyl-1-carboxylic [acide (1R, 2S)-2-octylcyclopropyl-1-carboxylique (II) et acide (1S, 2R)-2-octylcyclopropyl-1-carboxylique (III)] (27%).

### Analyses spectroscopiques :

### RMN 1H (200 MHz, CDCl3):

δ = 10,16 (br, s, 1 H), 1,45-1,75 (m, 3 H), 1,18-1,44 (m, 13 H), 0,91-1,14 (m, 2 H), 0,88 (t, J = 6,3 Hz, 3 H) ppm.

### RMN 13C (50 MHz, CDCl3) :

δ = 180,57, 32,67, 30,32 (x2), 30,09 (×2), 27,67, 23,93, 23,47, 18,85, 15,20, 14,90.

### MS (El, 70 eV) :

180 (2), 151 (5), 138 (17), 97 (40), 96 (30), 84 (34), 83 (39), 73 (100), 70 (33), 69 (43), 56 (37), 55 (64), 43 (47), 41 (52).

### Exemple 8 : Synthèse de chacun des énantiomères du 2-octylcyclopropyl-1-carboxylic acids 8-9

### Protocole général :

Ce protocole est illustré par la figure 2.

### Réactif et conditions

a) DHP, Amberlyst® 15, éther de pétrole, Température ambiante, 7h, 89%;
b) NaH, DMSO (4 eq.), THF, Température ambiante, 24h, puis 1-bromooctane, Température ambiante, 24h, 67% ;
c) Amberlyst® 15, Méthanol, 45°C, 15h, 95%;
d) LiAlH₄, THF, reflux 2h, 68% ;
e) H₂, Ni-P2, éthylènediamine, Méthanol, Température ambiante, 60h, 64% ;
f) Et₂Zn, CH₂l₂, hexane, -35°C → Température ambiante, 13h ;
g) (-)-10, CH₂Cl₂, -15°C, puis Zn(CH₂l)₂-DME, CH₂Cl₂, -15°C → Température ambiante, 15h ;
h) (+)-10, CH₂Cl₂, -15°C, puis Zn(CH₂l)₂-DME, CH₂Cl₂, -15°C → Température ambiante, 15h ;
i) Réactif de Jones, acétone, Température ambiante, 20h (rendements après deux étapes: (±)-8, 53%; (+)-8, 66%; (-)-8, 79%; (±)-9, 17%; (+)-9, 66%; (-)-9, 80%).

### Procédure détaillée :

### Exemple 8.1 : Synthèse de l'éther2-propynyloxytetrahydro-2H-pyran-2-yl[1] :

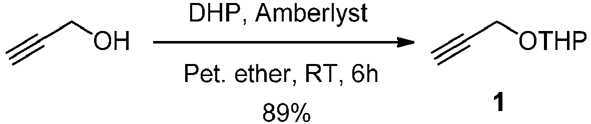

On prépare le mélange de 144 ml (139 g, 2,47 mol, 1 eq.) d'alcool propargylic (prop-2-yn-1-ol) et de 250 ml de 3,4-dihydropyrane (230 g, 2,74 mol, 1,11 eq.) dans de l'éther de pétrole (1,85 L). On ajoute alors 3,53g d'amberlyst® 15. La suspension est alors agitée à température ambiante pendant 6 heures, puis filtrée sur célite and évaporée sous pression réduite pour obtenir une huile brune.

On obtient après distillation 329,7g (89%) éther de 2-propynetetrahydro-2*H*-pyran-2-yl (1) sous la forme d'une huile incolore.

### Données spectrales :

### ¹H NMR (200 MHz, CDCl₃) :

*δ* = 1,48 - 1,85 (m, 6H), 2,40 (t, 1H), 3,48 - 3,58 (m, 1H), 3,77 - 3,82 (m, 1H), 4,24 (m, 2H), 4,80 (t, 1H).

### ¹³C NMR (50 MHz, CDCl₃) :

*δ* = 19,09, 25,43, 30,30, 54,09, 62,08, 74,09, 79,88, 96,93.
MS (El, 70 eV) : 85 (100), 82 (13), 57 (27), 56 (45), 55 (36), 53 (15), 43 (12), 41 (42), 39 (51).

### Exemple 8.2 : Synthèse de l'éther du 2-undecynyltetrahydro-2H-pyran-2-yl [2] :

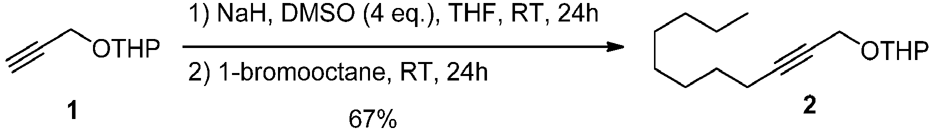

Une dispersion à 60% d'hydrure de sodium (27,16 g, 679 mmol, 2,32 eq.) est lavée 2 fois avec de l'éther de pétrole puis couverte avec 400 ml de tétrahydrofurane (THF) sec.

On ajoute à cette suspension 88 g de DMSO anhydre (80 ml, 1,12 mol, 3,85 eq.) sous agitation, puis 41 g d'éther de 2-propynetetrahydro-2*H*-pyran-2-yl [1] (40,6 ml, 290 mmol, 1 eq.) obtenu précédemment.

Après 24 heures d'agitation, à température ambiante, 141 g de 1-bromooctane (126 ml, 730 mmol, 2,5 eq.) sont ajoutés goutte à goutte durant environ une heure.

La température interne de la réaction atteint 38°C, puis décroit.

Le milieu réactionnel est agité pendant 48 h à température ambiante et est éventuellement refroidi par un bain de glace.

200 ml d'une solution aqueuse saturée d'hydrogénocarbonate de sodium sont alors ajoutés goutte à goutte. Puis on ajoute 200 ml d'eau.

Après décantation du mélange biphasique obtenu, la phase aqueuse est lavée 2 fois avec 200 ml d'éther de pétrole.

Les phases organiques réunies sont lavées avec de la saumure, séchées sur sulfate de magnésium et évaporées sous pression réduite. L'huile brun foncé obtenue est alors purifiée par distillation sous vide (0,46 mbars) pour obtenir de 49,5 g (67%) d'éther de 2-undecynyltetrahydro-2H-pyran-2-yl [2] sous la forme d'une huile jaune pale.

### Données spectrales :

### ¹H NMR (200 MHz, CDCl₃) :

δ = 0,88 (t, 3H), 1,20 - 1,97 (m, 18H), 2,15 - 2,31 (m, 2H), 3,46 - 3,69 (m, 1H), 3,78 - 3,86 (m, 1 H), 4,12 - 4,38 (m, 2H), 4,81 (1H, br t),

### ¹³C NMR (50 MHz, CDCl3) :

δ = 14,19, 18,92, 19,24, 22,76, 25,51, 28,72, 28,99, 29,20, 29,30, 30,41, 31,94, 54,74, 62,07, 75,81, 86,86, 96,70.
MS (El, 70 eV) : 101 (32), 95 (50), 93 (18), 85 (100), 81 (39), 79 (24), 67 (40), 55 (40), 43 (24), 41 (38).

### Exemple 8.3 : Synthèse du 2-undecynol [3] :

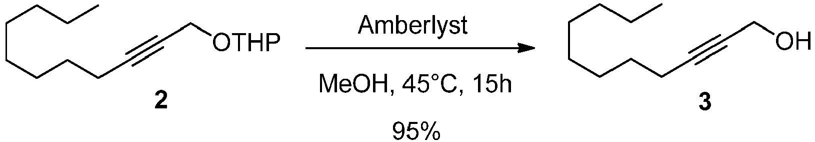

A une suspension de 73 mg d'amberlyst® H-15 dans 16 ml de méthanol, on ajoute 1,030 g d'éther de 2-undecynyltetrahydro-2H-pyran-2-yl [2] (4,08 mmol) obtenu précédemment. Ce mélange est agité à 45°C pendant 15h, filtré sur célite, évaporé sous pression réduite, et filtré sur un court tampon de gel de silice en utilisant un mélange 9/1 éther de pétrole / éther diéthylique pour rincer. Après évaporation des solvants, 0,651 g de 2-undecynol (3) (95%) sont obtenus sous la forme d'une huile légèrement jaune.

### Données spectrales :

### ¹H NMR (200 MHz, CDCl₃) :

δ = 0,88 (t, 3H), 1,22 - 1,61 (m, 12H), 2,15 - 2,26 (m, 2H), 4,20 - 4,29 (m, 2H),

### ¹³C NMR (50 MHz, CDCl₃) :

*δ* = 14,22, 18,86, 22,78, 28,73, 29,01, 29,23, 29,31, 31,96, 51,53, 78,38, 86,78.
MS (El, 70 eV) : 93 (56), 83 (45), 81 (68), 79 (63), 70 (64), 67 (78), 55 (100), 43 (52), 41 (93), 39 (48).

### Exemple 8.4 : Synthèse du (Z)-undec-2-en-1-ol[4] :

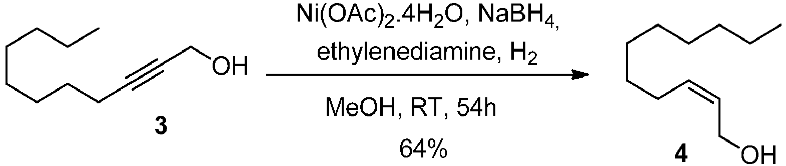

2,69 g (10,8 mmol, 0,26 eq.) d'acétate de Nickel(II) tétrahydrate (Ni(OAc)₂, 4H₂O) sont dissouts dans 66 mL de méthanol par agitation sous argon et la solution obtenue est alors refroidie dans un bain de glace.

457 mg de borohydrure de sodium (12,1 mmol, 0,29 eq,) sont alors ajoutés rapidement en une fois et le bain de glace est retiré.

Après 10 minutes d'agitation, 1,22 g de 1,2-éthylènediamine (20,3 mmol, 0,48 eq) sont ajoutés, suivi après 10 minutes par l'ajout de 7,07 g de 2-undecynol [3] (42 mmol, 1 eq.). L'atmosphère du flacon est alors purgée et maintenue sous une légère surpression d'hydrogène à l'aide d'un ballon.

Le mélange réactionnel est alors mélangé pendant 54h, et la réaction est suivie par GC-MS.

Pour assurer la conversion complète du matériel de départ deux portions additionnelles de Ni(OAc)₂, 4H₂O/ borohydrure de sodium /1,2-ethylenediamine sont ajoutées séquentiellement, jusqu'à une quantité additionnelle totale de 563 mg (2,26 mmol, 0,05 eq.) de Ni(OAc)₂, 4H₂O, 139 mg (3,69 mmol, 0,09 eq.) de borohydrure de sodium et 352 mg (5,86 mmol, 0,14 eq.) de 1,2-éthylènediamine.

Le mélange réactionnel est alors filtré sur Célite et le solide est lavé à l'aide de dichlorométhane.

Le filtrat ainsi obtenu est évaporé et 100 ml d'éther diéthylique et 100ml d'eau sont ajoutés à ce résidu.

Après décantation la phase aqueuse d'un bleu profond est lavée avec de l'éther diéthylique (3 × 50 ml), et les phases organiques combinées sont alors lavées avec de la saumure, séchées sur sulfate de magnésium et évaporées sous pression réduite. L'huile verte est alors purifiée par chromatographie sur colonne de gel de silice (éther de pétrole / éther diéthylique (95/5 → 9/1)) jusqu'à obtention de 4,32 g (60%) (*Z*)-undec-2-en-1-ol [4] sous la forme d'une huile incolore.

### Données spectrales :

### ¹H NMR (200 MHz, CDCl3) :

*δ* = 5.55 (m, 2 H), 4,17 (d, 2 H), 2,05 (q, 2 H), 1,20-1,45 (m, 12 H), 0,88 (t, 3 H) ppm.

### ¹³C NMR (50 MHz, CDCl₃) :

δ = 133,27, 128,43, 58,65, 31,99, 29,73, 29,57, 29,39, 29,35, 27,54, 22,78, 14,21 ppm.
MS (EI, 70 eV) : 170 (M⁺, <1), 95 (28), 82 (43), 81 (31), 69 (27), 68 (33), 67 (34), 57 (100), 55 (41), 43 (43), 41 (50).
IR (neat) νₘₐₓ 3315, 2923, 2854, 1464, 1033 cm⁻¹.

### Exemple 8.5 : Synthèse du (E)-undec-2-en-1-ol [SI-5] :

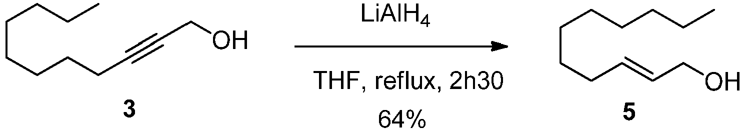

6,9 g de SI-3 (41 mmol, 1,00 eq.) sont ajoutés à 3,53 g d'une suspension de tétrahydruroaluminate de lithium, (LiAlH₄) (93 mmol, 2,27 eq.) dans du THF anhydre (650 mL), et le mélange est porté à reflux pendant 2h30.

Après refroidissement dans un bain de glace, le mélange réactionnel est alors quenché par addition prudente d'une solution saturée de sulfate de sodium (12 mL), suivi par l'ajout de 14g de sulfate de sodium anhydre. Le mélange est alors agité pendant 2h, la suspension grise résultante est alors filtré sur Célite et le solide est lavé de manière exhaustive avec de l'éther diéthylique. Après évaporation du solvant, l'huile brute est purifiée par chromatographie sur colonne de gel de silice (éther de pétrole / éther diéthylique (95/5 → 9/1)) jusqu'à obtention de 4,47 g de (*E*)-undec-2-en-1-ol [SI-5] (64%) sous la forme d'une huile incolore.

### Données spectrales :

### ¹H NMR (200 MHz, CDCl₃):

*δ* = 5,48-5,74 (m, 2 H), 4,02 (d, *J* = 4,6 Hz, 2 H), 2,41 (br, s, 1 H), 2,00 (q, *J* = 6,9 Hz, 2 H), 1,15-1,40 (m, 12 H), 0,85 (t, *J* = 6,2 Hz, 3 H) ppm.

### ¹³C NMR (50 MHz, CDCl₃):

δ = 133,32, 128,92, 63,60, 32,30, 31,96, 29,55, 29,36, 29,29, 29,24, 22,73, 14,14 ppm.
MS (EI, 70 eV): 170 (M⁺, <1), 82 (43), 81 (34), 69 (32), 68 (36), 67 (41), 57 (100), 55 (51), 54 (33), 43 (49), 41 (63).
IR (neat) νₘₐₓ 3315, 2923, 2854, 1464, 1033 cm⁻¹.

### Exemple 8.6 : Synthèse du (±)-trans-2-octylcyclopropyl-1-methanol [(±)-6] :

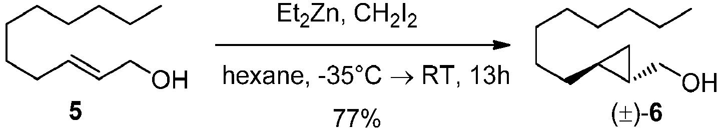

A une solution de 332 mg d'(*E*)-undec-2-en-1-ol (5) (1,95 mmol, 1 eq.) dans 10 ml d'hexane, sont ajoutés goutte à goutte 10 mL d'une solution à 1M de diéthylzinc (10 mmol, 5,1 eq.) dans de l'hexane à -50°C, suivie par 5,28 g de diiodométhane (19,7 mmol, 10 eq.).

Le mélange réactionnel est lentement réchauffé à température ambiante et agité pendant 18h, puis de nouveau refroidi à -40°C, quenché par l'addition goutte à goutte d'une solution saturée de chlorure d'ammonium (20 mL). Le mélange est alors laissé à réchauffer jusqu'à température ambiante sous agitation. Le mélange est alors décanté, et la phase aqueuse est lavées avec 2×25 ml d'éther diéthylique. Les phases organiques combinées sont lavées avec de la saumure, séchées sur sulfate de magnésium et évaporées sous pression réduite pour obtenir une huile orange (788 mg) qui est alors purifiée par chromatographie sur colonne de gel de silice (éther de pétrole / éther diéthylique (98/2 → 8/2)) jusqu'à obtention de 275 mg de (±)-*trans*-2-octylcyclopropyl-1-methanol [(±)-6] (77%) sous la forme d'une huile incolore.

### Données spectrales :

### ¹H NMR (200 MHz, CDCl₃):

*δ* = 3,28 - 3,48 (m, 2 H), 2,27 (br, s, 1 H), 1,10 - 1,40 (m, 14 H), 0,88 (t, 3 H), 0,70 - 0,85 (m, 1 H), 0,45 - 0,63 (m, 1 H), 0,18 - 0,38 (m, 2 H) ppm.
¹³C NMR (50 MHz, CDCl₃): *δ* = 67,06, 33,71, 31,98, 29,71, 29,67, 29,54, 29,41, 22,74, 21,14, 17,20, 14,15, 9,99 ppm.
MS (EI, 70 eV) : 143 (34), 83 (67), 82 (38), 81 (41), 69 (100), 68 (34), 67 (46), 57 (66), 55 (70), 43 (49), 41 (64).
IR (neat) νₘₐₓ 3300, 2922, 2852, 1464, 1032 cm⁻¹

### Exemple 8.9 : Synthèse de l'acide (±)-trans-2-octylcyclopropyl-1-carboxylique [(±)-8] :

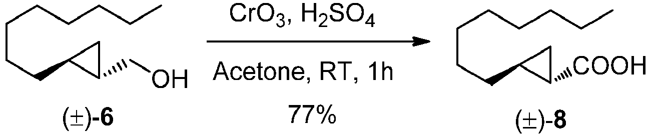

A une solution de 130 mg de (±)-*trans*-2-octylcyclopropyl-1-methanol [(±)-6] (0,71 mmol, 1 eq.) dans 3 ml d'acétone, on ajoute goutte à goutte une solution 322 mg de trioxyde de chrome (3,22 mmol, 4,6 eq.) dans 1,08 mL d'acide sulfurique 36% à 0°C. Le mélange réactionnel est lentement réchauffé jusqu'à température ambiante et agité pendant 1h. 5 ml d'eau sont alors ajoutés et le mélange est évaporé, additionné de 25 ml d'eau et lavé avec 2 × 125 ml de dichlorométhane. Les phases organiques combinées sont alors lavées à la saumure, séchées sur sulfate de magnésium et évaporées sous pression réduite jusqu'à obtention d'un résidu qui est alors purifié purifiée par chromatographie sur colonne de gel de silice (éther de pétrole / éther diéthylique (9/1 → 8/2)) pour obtenir 103 mg d'acide (±)-*trans*-2-octylcyclopropyl-1-carboxylique [(±)-8] (73%).

### Données spectrales :

### ¹H NMR (200 MHz, CDCl₃) :

*δ* = 11,73 (br, s, 1 H), 1,14 - 1,48 (m, 17 H), 0,88 (t, 3 H), 0,70 - 0,82 (m, 1 H) ppm,

### ¹³C NMR (50 MHz, CDCl₃) :

*δ* = 181,54, 33,18, 32,02, 29,67, 29,42, 29,41, 29,19, 24,19, 22,81, 20,27, 16,52, 14,23 ppm.
MS (El, 70 eV) : 180 (1), 151 (5), 138 (15), 97 (39), 84 (34), 83 (39), 73 (100), 70 (35), 69 (51), 56 (46), 55 (79), 43 (66), 41 (72).
IR (neat) νₘₐₓ 2917, 2850, 1687, 1456, 1431, 1222, 874 cm⁻¹

### Exemple 8.10 : Synthèse du (±)-cis-2-octylcyclopropyl-1-methanol [7] :

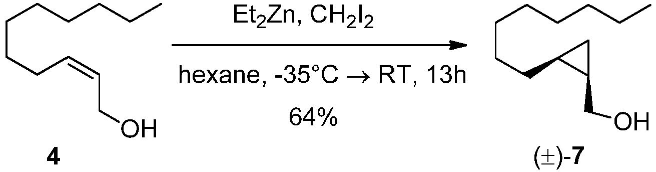

La même procédure que ci-dessus appliquée au (*Z*)-undec-2-en-1-ol[4] conduit à l'obtention de (±)-*cis*-2-octylcyclopropyl-1-methanol [7] (64%) sous la forme d'une huile incolore.

### Données spectrales :

¹H NMR (200 MHz, CDCl₃): *δ* = 3,47 - 3,74 (m, 2 H), 1,00 - 1,48 (m, 16 H), 0,87 (t, 3 H), 0,62 - 0,78 (m, 1 H), -0,10 - 0,01 (q, 1 H) ppm.
MS (EI, 70 eV) : 143 (30), 83 (66), 82 (35), 81 (39), 69 (100), 68 (30), 67 (44), 57 (56), 55 (65), 43 (45), 41 (59).
IR (neat) νₘₐₓ 3300, 2923, 2853, 1465, 1033 cm⁻¹

### Exemple 8.11 : Synthèse de l'acide (±)-cis-2-octylcyclopropyl-1-carboxylique [(±)-9] :

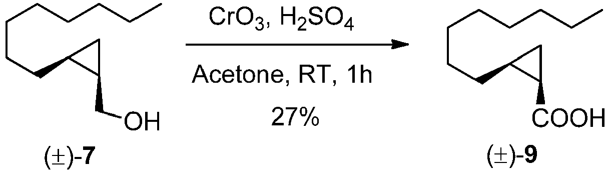

La même procédure que ci-dessus appliquée au *cis*-2-octylcyclopropyl-1-methanol [(±)-7] conduit à l'obtention de l'acide (±)-*cis*-2-octylcyclopropyl-1-carboxylique [(±)-9] (27%) sous la forme d'une huile incolore.

### Données spectrales :

### ¹H NMR (200 MHz, CDCl₃):

*δ* = 10,16 (br. s, 1 H), 1,45 - 1,75 (m, 3 H), 1,18 - 1,44 (m, 13 H), 0,91 -1,14 (m, 2 H), 0,88 (t, 3 H) ppm.
¹³C NMR (50 MHz, CDCl₃): *δ* = 180,57, 32,67, 30,32 (×2), 30,09 (×2), 27,67, 23,93, 23,47, 18,85, 15,20, 14,90.
MS (EI, 70 eV) : 180 (2), 151 (5), 138 (17), 97 (40), 96 (30), 84 (34), 83 (39), 73 (100), 70 (33), 69 (43), 56 (37), 55 (64), 43 (47), 41 (52).
IR (neat) νₘₐₓ 2923, 2854, 1693, 1033 cm⁻¹

### Exemple 8.12 : Synthèse du (1S, 2S)-trans-2-octylcyclopropyl-1-méthanol [(1S, 2S)-6] :

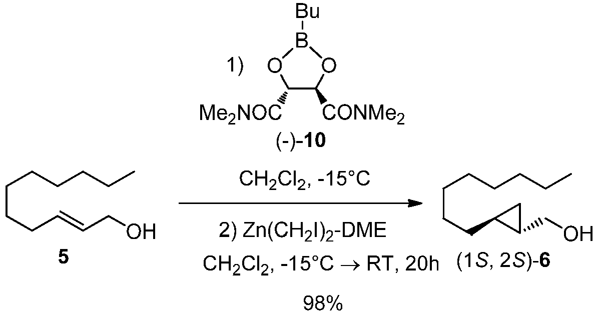

En premier, une solution de complexe DME de bisiodozinc [Zn(CH₂l)₂·DME] est préparée par addition de diiodométhane (19,2 ml, 63,8 g, 238 mmol, 21,1 eq.) en 1h (à l'aide d'une seringue à pompe) à une solution 1M de diéthylzinc (120 ml, 120 mmol, 10.64 eq.) dans un mélange de dichlorométhane sec (240 ml) et de diméthoxyéthane (12,4 ml) à -15°C.

La solution laiteuse résultante est maintenue à froid par un bain de glace.

Parallèlement, 1,92 g d'(*E*)-undec-2-en-1-ol (5) (11,3 mmol, 1 eq.) et 3,60 g de (4*R*, 5*R*)-2-butyl-N,N,N',N'-tetramethyl[1,3,2]dioxaborolane-4,5-dicarboxamide [(-)-10] (13,3 mmol, 1,18 eq.) sont dilués dans 60 ml de dichlorométhane, et le mélange est refroidi à -14°C. La solution de complexe de Zn(CH₂l)₂·DME préalablement préparée est alors lentement canulée dans le mélange en environ 12 min., et agitée à une température comprise entre -12°C et -18°C pendant 4h, puis le tout est laissé revenir lentement à la température ambiante sous agitation pendant la nuit. Le mélange est alors quenché par ajout goutte à goutte d'une solution saturée de chlorure d'ammonium (80 mL) sous agitation. Le mélange est alors décanté et la phase aqueuse est lavée avec 3×60 ml d'éther diéthylique. Les phases organiques combinées sont lavées successivement avec des solutions de 5M d'hydroxyde de potassium, 10% d'acide hydrochlorique, saturée en bicarbonate de sodium et éventuellement deux fois avec de la saumure. On sèche alors sur sulfate de magnésium and évapore sous pression réduite jusqu'à obtenir une huile jaune qui est alors purifiée par filtration sur un court tampon de gel de silice (20 g). La première portion du filtrat (en utilisant 80 ml d'éther de pétrole) est jetée et le produit est élué avec 150 ml d'éther de pétrole / éther diéthylique (2/1) pour obtenir après évaporation 2,08g de (1*S*, 2S)-*trans*-2-octylcyclopropyl-1-methanol [(1*S*, 2*S*)-6] (98%) sous la forme d'une huile jaune pale.

Les données spectrales sont identiques à celles du [(±)-6].

### Exemple 8.13 : Synthèse de l'acide (+)-trans-2-octylcyclopropyl-1-carboxylique [(+)-8] :

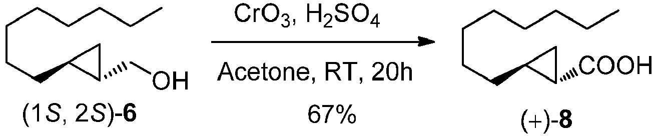

La procédure d'oxydation utilisée pour la synthèse du [(±)-8] sont appliquées au (1*S*, 2*S*)-6, avec un temps de réaction de 20h, pour obtenir du (+)-(1*S*, 2*S*)-8 (67%).

Les données spectrales sont identiques à celles du [(±)-8].

### Exemple 8.14 : Synthèse du (1R, 2R)-trans-2-octylcyclopropyl-1-méthanol [(1R, 2R)-6] :

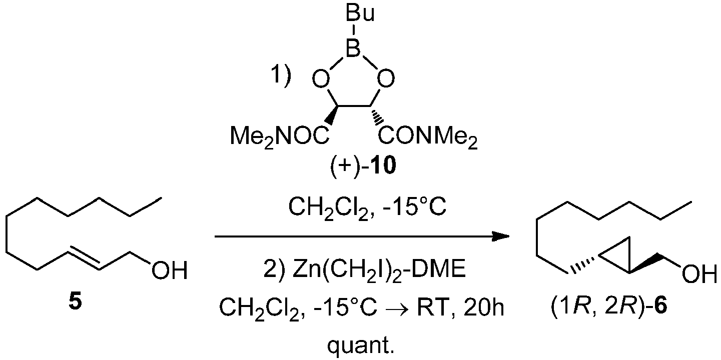

La procédure de cyclopropanation asymétrique utilisée pour la synthèse du (1*S*, 2*S*)-6 est appliquée à [5], avec [(+)-10] utilisé comme auxiliaire chiral, et conduit au [(1*R,* 2*R*)-6] (quant.).

Les données spectrales sont identiques à celles du 6.

### Exemple 8.15 : Synthèse de l'acide (-)-trans-2-octylcyclopropyl-1-carboxylique [(-)-8] :

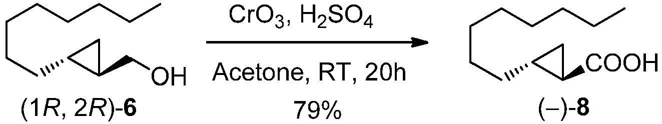

La procédure d'oxydation utilisée pour le [(±)-8] est appliquée à [(1*R,* 2*R*)-6], avec un temps de réaction de 20h, et conduite au [( )-(1*R,* 2*R*)-8] (79%).

Les données spectrales sont identiques à celles du [(±)-8].

### Exemple 8.16 : Synthèse du (1S, 2R)-cis-2-octylcyclopropyl-1-méthanol [(1S, 2R)-7] :

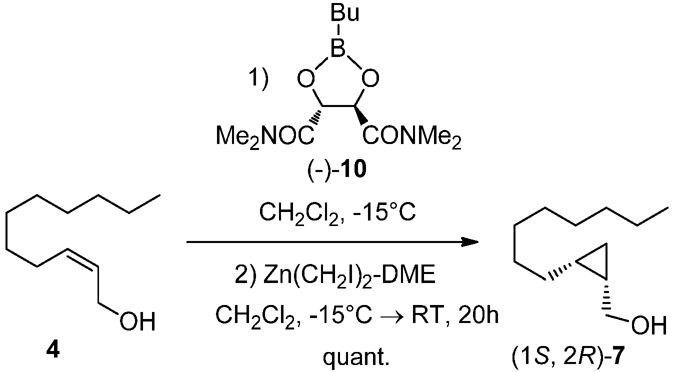

La procédure de cyclopropanation asymétrique utilisée pour la synthèse du [(1*S*, 2*S*)-6] est appliquée à [4] et conduit au [(1*S*, 2*R*)-7] (quant.).

Les données spectrales sont identiques à celles du [(±)-7].

### Exemple 8.17 : Synthèse de l'acide (+)-cis-2-octylcyclopropyl-1-carboxylique [(+)-9] :

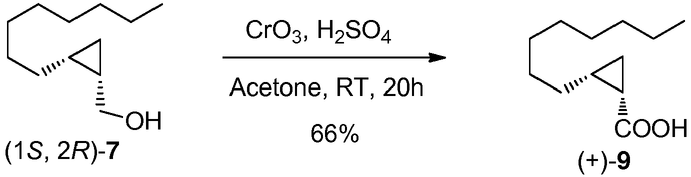

La procédure d'oxydation utilisée pour le [(±)-8] est appliquée au [(1*S*, 2*R*)-7], avec un temps de réaction de 20h, et conduit au [(+)-(1*S*, 2*R*)-9] (66%).

Les données spectrales sont identiques à celles du [(±)-9].

### Exemple 8.18 : Synthèse du (1R, 2S)-cis-2-octylcyclopropyl-1-méthanol [(1R, 2S)-7] :

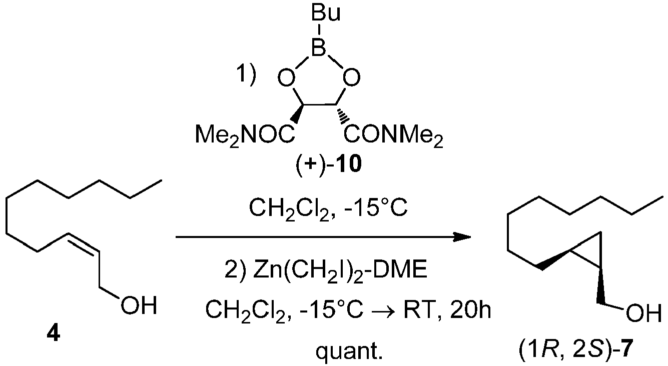

La procédure de cyclopropanation asymétrique utilisée pour la synthèse du [(1*S*, 2*S*)-6] est appliquée à [4] avec [(+)-10] utilisé comme auxiliaire chiral, et conduit au [(1*R*, 2*S*)-7] (quant.).

Les données spectrales sont identiques à celles du [(±)-7].

### Exemple 8.19 : Synthèse de l'acide (-)-cis-2-octylcyclopropyl-1-carboxylique [(-)-9] :

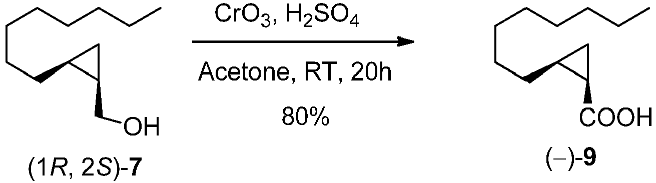

La procédure d'oxydation utilisée pour le [(±)-8] est appliquée au [(1*R*, 2*S*)-7], avec un temps de réaction de 20h, et conduit au [(-)-(1*R*, 2*S*)-9] (80%).

Les données spectrales sont identiques à celles du [(±)-9].

## Revendications

1. Isomère trans ou cis de l'acide 2-octylcyclopropyl-1-carboxylique, isolé, choisi parmi
▪ l'acide (1R, 2S)-2-octylcyclopropyl-1-carboxylique de formule II 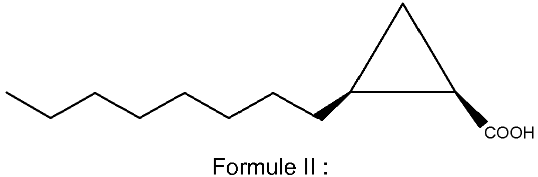
▪ l'acide (1S, 2R)-2-octylcyclopropyl-1-carboxylique de formule III 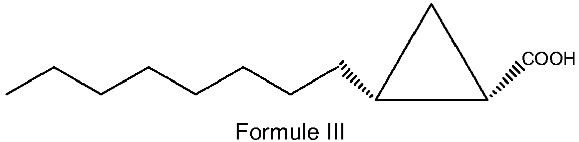
▪ l'acide (1R, 2R)-2-octylcyclopropyl-1-carboxylique de formule IV 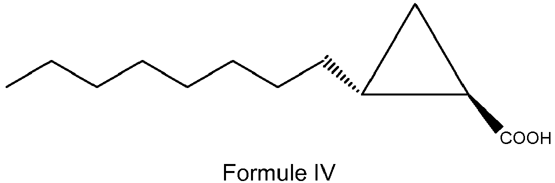
▪ l'acide (1S, 2S)-2-octylcyclopropyl-1-carboxylique de formule V ; et

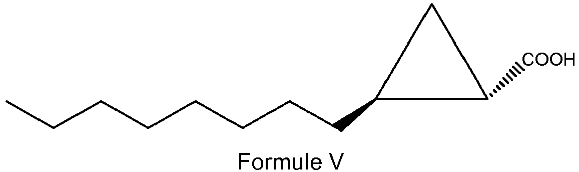

2. Isomères trans ou cis de l'acide 2-octylcyclopropyl-1-carboxylique, **caractérisés en ce qu'**ils sont sous la forme d'un mélange en toutes proportions choisi parmi les mélanges
- Isomères de formule II, III et IV,
- Isomères de formule II, III et V,
- Isomères de formule II, IV et V,
- Isomères de formule III, IV et V,
- Isomères de formule II et III,
- Isomères de formule II et IV
- Isomères de formule II et V,
- Isomères de formule III et IV,
- Isomères de formule III et V.

3. Isomère de formule II selon la revendication 1 isolé.

4. Isomère de formule III selon la revendication 1 isolé.

5. Isomère de formule IV selon la revendication 1 isolé.

6. Isomère de formule V selon la revendication 1 isolé.

7. Composition comprenant au moins un isomère isolé de l'acide 2-octylcyclopropyl-1-carboxylique tel que décrit dans l'une quelconque des revendications 3 à 6.

8. Composition comprenant au moins un mélange d'isomères tel que décrit à la revendication 2.

9. Composition selon l'une quelconque des revendications 8 ou 9, **caractérisée en ce qu'**il s'agit d'une composition cosmétique, pharmaceutique, vétérinaire, phytosanitaire, hygiénique, de nettoyage, de lavage.

10. Utilisation d'un isomère isolé de l'acide 2-octylcyclopropyl-1-carboxylique ou d'un mélange desdits isomères tels que décrits dans l'une quelconque des revendications 2 à 6 à titre d'agent parfumant.

## Patentansprüche

1. Isoliertes trans- oder cis-Isomer von 2-Octylcyclopropyl-1-carbonsäure, ausgewählt aus
▪ (1R, 2S)-2-Octylcyclopropyl-1-carbonsäure der Formel II 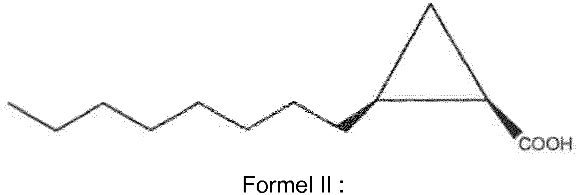
▪ (1S, 2R)-2-Octylcyclopropyl-1-carbonsäure der Formel III 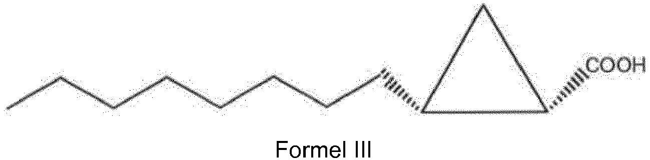
▪ (1R, 2R)-2-Octylcyclopropyl-1-carbonsäure der Formel IV 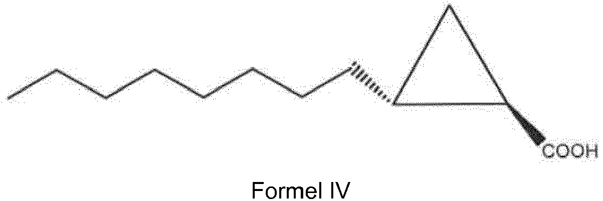
▪ (1S, 2S)-2-Octylcyclopropyl-1-carbonsäure der Formel V; und

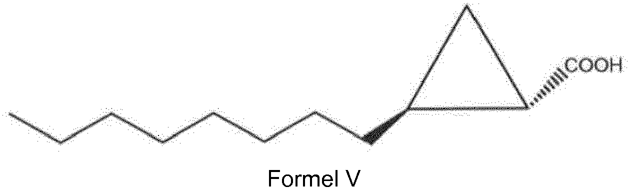

2. Trans- oder cis-Isomere von 2-Octylcyclopropyl-1-carbonsäure, **dadurch gekennzeichnet, dass** sie in Form einer Mischung in einem beliebigen Verhältnis vorliegen, ausgewählt aus den folgenden Mischungen:
- Isomere der Formeln II, III und IV,
- Isomere der Formeln II, III und V,
- Isomere der Formeln II, IV und V,
- Isomere der Formeln III, IV und V,
- Isomere der Formeln II und III,
- Isomere der Formeln II und IV
- Isomere der Formeln II und V,
- Isomere der Formeln III und IV,
- Isomere der Formeln III und V.

3. Isoliertes Isomer der Formel II nach Anspruch 1.

4. Isoliertes Isomer der Formel III nach Anspruch 1.

5. Isoliertes Isomer der Formel IV nach Anspruch 1.

6. Isoliertes Isomer der Formel V nach Anspruch 1.

7. Zusammensetzung, die mindestens ein isoliertes Isomer von 2-Octylcyclopropyl-1-carbonsäure wie in einem der Ansprüche 3 bis 6 beschrieben umfasst.

8. Zusammensetzung, die mindestens eine Mischung von Isomeren wie in Anspruch 2 beschrieben umfasst.

9. Zusammensetzung nach Anspruch 8 oder 9, **dadurch gekennzeichnet, dass** sie eine kosmetische, pharmazeutische, veterinäre, phytosanitäre, hygienische, reinigende, waschende Zusammensetzung ist.

10. Verwendung eines isolierten Isomers von 2-Octylcyclopropyl-1-carbonsäure oder einer Mischung dieser Isomere wie in einem der Ansprüche 2 bis 6 beschrieben als Duftstoff.

## Claims

1. Isolated trans or cis isomer of 2-octylcyclopropyl-1-carboxylic acid, selected from
▪ (1R, 2S)-2-octylcyclopropyl-1-carboxylic acid of formula II 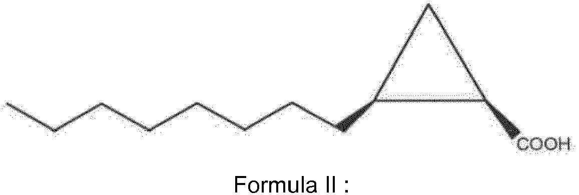
▪ (1S, 2R)-2-octylcyclopropyl-1-carboxylic acid of formula III 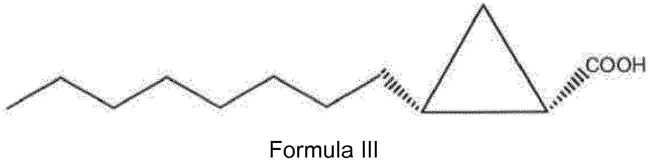
▪ (1R, 2R)-2-octylcyclopropyl-1-carboxylic acid of formula IV 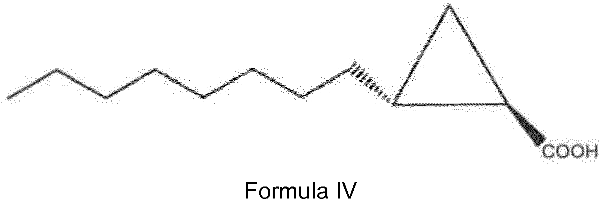
▪ (1S, 2S)-2-octylcyclopropyl-1-carboxylic acid of formula V; and

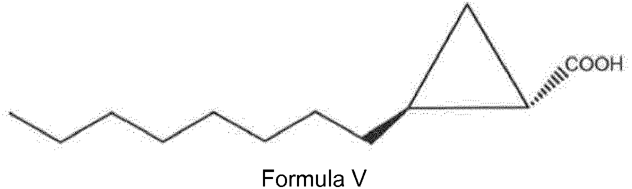

2. Trans or cis isomers of 2-octylcyclopropyl-1-carboxylic acid, **characterized in that** they are in the form of a mixture in any proportion selected from the mixtures
- Isomers of formula II, III and IV,
- Isomers of formula II, III and V,
- Isomers of formula II, IV and V,
- Isomers of formula III, IV and V,
- Isomers of formula II and III,
- Isomers of formula II and IV
- Isomers of formula II and V,
- Isomers of formula III and IV,
- Isomers of formula III and V.

3. Isolated isomer of formula II according to claim 1.

4. Isolated isomer of formula III according to claim 1.

5. Isolated isomer of formula IV according to claim 1.

6. Isolated isomer of formula V according to claim 1.

7. Composition comprising at least one isolated isomer of 2-octylcyclopropyl-1-carboxylic acid as described in any one of claims 3 to 6.

8. Composition comprising at least one mixture of isomers as described in claim 2.

9. Composition according to any one of claims 8 or 9, **characterized in that** it is a cosmetic, pharmaceutical, veterinary, phytosanitary, hygiene, cleaning, washing composition.

10. Use of an isolated isomer of 2-octylcyclopropyl-1-carboxylic acid or of a mixture of said isomers as described in any one of claims 2 to 6 as a perfuming agent.
